(19) Europäisches Patentamt
European Patent Office
Office européen des brevets

(11) Veröffentlichungsnummer: **0 085 181**
**B1**

(12) # EUROPÄISCHE PATENTSCHRIFT

(45) Veröffentlichungstag der Patentschrift: **03.10.84**

(51) Int. Cl.³: **C 07 C 148/00**, C 07 C 149/40

(21) Anmeldenummer: **82111925.2**

(22) Anmeldetag: **23.12.82**

(54) **Verfahren zur Herstellung von o,o'-Dithiodibenzoesäuren.**

(30) Priorität: **22.01.82 DE 3201904**

(43) Veröffentlichungstag der Anmeldung:
**10.08.83 Patentblatt 83/32**

(45) Bekanntmachung des Hinweises auf die Patenterteilung:
**03.10.84 Patentblatt 84/40**

(84) Benannte Vertragsstaaten:
**BE CH DE FR GB IT LI NL**

(56) Entgegenhaltungen:
**BEILSTEINS HANDBUCH DER ORGANISCHEN CHEMIE, 4. Auflage, Band X 1927, VERLAG VON JULIUS SPRINGER, Berlin**
**CHEMISCHE BERICHTE, "Verfahren zur Herstellung oramatischer Sulfonsäurechloride, eine neue Modifikation der sandmeyerschen Reaktion", Band 90, Nr. 6, 1957 VERLAG CHEMIE, Weinheim/Bergstr. Seiten 841-852**
**Chemical Abstracts Band 63, Nr. 1, 5. Juli 1965 Columbus, Ohio, USA Spalte 541c & IN. - A - 79073**
**Chemical Abstracts Band 45, Nr. 18, 25. September 1951, Columbus, Ohio, USA C. van der Stelt et al. "Synthesis of 2-mercapto-4-aminobenzoic acid" Spalte 7983al,**

(73) Patentinhaber: **BASF Aktiengesellschaft, Carl-Bosch-Strasse 38, D-6700 Ludwigshafen (DE)**

(72) Erfinder: **Jaedicke, Hagen, Dr., Budapester Strasse 49, D-6700 Ludwigshafen (DE)**
Erfinder: **Tonne, Peter, Dr., Triftbrunnenweg 63, D-6730 Neustadt (DE)**

**EP 0 085 181 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Herstellung von o,o'-Dithiodibenzoesäuren durch Umsetzung von diazotierten Anthranilsäuren bzw. -estern mit Schwefeldioxid in Gegenwart von Säure, Wasser und eines Kupfersalzes und gewünschtenfalls in Gegenwart von Alkalijodid in einer 1. Stufe bei einer Temperatur von 20 bis 50°C und dann in einer 2. Stufe bei einer Temperatur von 80 bis 100°C.

Man stellt die o,o'-Dithiodibenzoesäure her, indem das Diazoniumsalz der Anthranilsäure in wässeriger Natriumdisulfidlösung zersetzt wird („Org. Synth. Coll.", vol. II, S. 580-583 [1943]). Bei diesem Verfahren erfordert das Arbeiten mit Sulfid und elementarem Schwefel beträchtlichen Aufwand, da Abwasser und Abluft vom giftigen Schwefelwaserstoff freigehalten werden müssen. Der Erfindung lag daher die Aufgabe zugrunde, ein Herstellverfahren für die o,o'-Dithiodibenzoesäure zu entwickeln, das die beschriebenen Nachteile vermeidet.

Meerwein, „Chem. Ber.", Bd. 90 (1957), S. 847, zeigt, dass sich diazotierte Anthranilsäure mit Schwefeldioxid in Eisessig in 51% Ausbeute zur o,o'-Dithiodibenzoesäure umsetzt. Bei grosstechnischer Ausführung geht die Essigsäure in der Regel verloren.

Es wurde nun gefunden, dass man o,o'-Dithiodibenzoesäuren der Formel

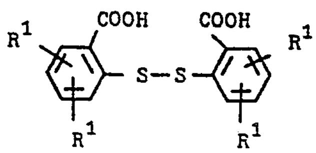

I

worin die einzelnen Reste $R^1$ gleich oder verschieden sein können und jeweils ein Wasserstoffatome in Halogenatom, eine Nitrogruppe, eine Gruppe $-SO_3H$ oder einen aliphatischen Rest bedeuten, durch Umsetzung von diazotierten Anthranilsäurederivaten mit Schwefeldioxid in Gegenwart von Säure, Wasser und Katalysatoren zur Zerstörung der Diazoniumsalze vorteilhaft erhält, wenn man ein Diazoniumsalz von Anthranilsäuren der Formel

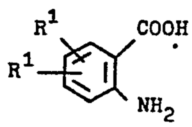

II

oder ein Diazoniumsalz von Anthranilsäureestern der Formel

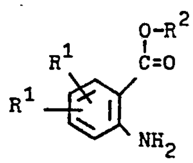

III

worin $R^1$ die vorgenannte Bedeutung besitzt und $R^2$ einen aliphatischen Rest bedeutet, in Gegenwart eines Kupfersalzes und gewünschtenfalls in Gegenwart von Alkalijodid in einer 1. Stufe bei einer Temperatur von 20 bis 50° C und dann in einer 2. Stufe bei einer Temperatur von 80 bis 100°C umsetzt.

Die Umsetzung kann, für den Fall der Verwendung des Diazoniumchlorids der Anthranilsäure, durch folgende Formeln wiedergegeben werden

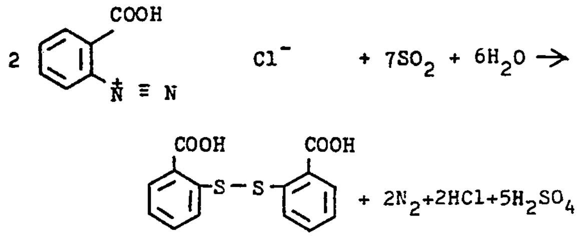


Im Vergleich zu den bekannten Verfahren liefert das Verfahren nach der Erfindung auf einfacherem und billigerem Wege o,o'-Dithiodibenzoesäuren in besserer Ausbeute und Reinheit. Im Hinblick auf das Sulfid und elementaren Schwefel verwendende Verfahren ist es wesentlich umweltfreundlicher, da kein Schwefelwasserstoff, elementarer Schwefel und Polysulfide und entsprechend keine wesentlichen Abluftprobleme entstehen. Ebenfalls werden Filtrationsschwierigkeiten durch elementaren Schwefel vermieden. Keine organischen Stoffe, z.B. Eisessig, gehen verloren. Alle diese vorteilhaften Ergebnisse sind im Hinblick auf den Stand der Technik überraschend.

Die Diazoniumsalze der Ausgangsstoffe II und III können mit Schwefeldioxid in stöchiometrischer Menge, vorteilhaft in einer Menge von 3,5 bis 10, insbesondere 3,5 bis 4,5 mol $SO_2$ je Mol Ausgangsstoff II bzw. III umgesetzt werden. Bevorzugte Ausgangsstoffe II und III und dementsprechend bevorzugte Endstoffe I sind solche, in deren Formeln die einzelnen Reste $R^1$ gleich oder verschieden sein können und jeweils ein Wasserstoffatom, eine Nitrogruppe, eine Gruppe $-SO_3H$ oder ein Bromatom oder insbesondere ein Chloratom, einen Alkylrest mit 1 bis 6 C-Atomen bedeuten, $R^2$ einen Alkylrest mit 1 bis 8 C-Atomen bezeichnet. Die vorgenannten Reste können noch durch unter den Reaktionsbedingungen inerte Gruppen, z.B. Alkylgruppen oder Alkoxygruppen mit 1 bis 4 C-Atomen substituiert sein. Werden die Ausgangsstoffe III umgesetzt, so werden während der Zersetzung des Diazoniumsalzes auch die Estergruppen zerstört, so dass Endstoff I ebenfalls erhalten wird.

So kommen beispielsweise folgende Ausgangsstoffe II in Frage: das Diazoniumsalz der Anthranilsäure oder das Diazoniumsalz von in 3,4,5,6-Stellung einfach oder in 3,4-, 3,5-, 3,6-, 4,5-, 5,6-Stellung gleich oder unterschiedlich zweifach durch Brom, Chlor, Methyl-, Ethyl-, Propyl-, Isopropyl-, Butyl-, sek.-Butyl-, Isobuyl-, tert.-Butyl-Gruppe substituierte Anthranilsäuren.

Auch folgende Ausgangsstoffe III kommen beispielsweise in Betracht: das Diazoniumsalz der unsubstituierten oder durch vorgenannte Substituenten einfach oder zweifach substituierte Anthranilsäuremethylester; entsprechende Diazoniumsalze unsubstituierter oder in vorgenannter Weise substituierter Ethyl-, Propyl-, Isopropyl-, Butyl-, sek.-Butyl-, Isobutyl-, tert.-Butylester.

Die Diazotierung wird in der üblichen Weise durchgeführt, z.B. setzt man das Ausgangsmaterial in üblicher Weise mit Natriumnitrit in wässeriger salzsaurer Lösung bei 0 bis 10°C, z.B. Während 5 bis 70 min um. Während zweckmässig äquimolare Mengen Anthranilsäure und Nitrit eingesetzt werden, kann die Salzsäure in weiten Grenzen schwanken. Bei grosstechnischer Herstellung setzt man jedoch zweckmässig von 1 bis 3 mol HCl, vorteilhaft 1 bis 2 mol HCl/mol Anthranilsäure ein. Entsprechend können anstelle von Salzsäure auch andere Säuren eingesetzt werden, wie Schwefelsäure oder Phosphorsäure. Besonders bevorzugt sind Diazoniumchloride und -sulfate. Durch Zugabe von Harnstoff werden dann zweckmässig Reste von Nitrit zerstört.

Die Umsetzung wird in 2 Stufen, in der 1. Stufe bei einer Temperatur von 20 bis 50, vorzugsweise von 30 bis 40°C, in der 2. Stufe bei einer Temperatur von 80 bis 100, vorzugsweise von 90 bis 100°C, drucklos oder unter Druck, kontinuierlich oder diskontinuierlich durchgeführt. Zweckmässig gibt man die fertige Diazotierlösung direkt in die 1. Stufe der erfindungsgemässen Umsetzung. 60 bis 90, insbesondere 70 bis 80 Gew.-% Wasser, bezogen auf die Gewichtsmenge Ausgangsstoff II oder III, sind vorteilhaft bei der Diazotierung sowie in beiden Stufen der Umsetzung. Ebenfalls ist ein Verhältnis von 0,5 bis 5 mol Säure, bezogen auf 1 mol Ausgangsstoff II oder III für Stufen 1 und 2 zweckmässig.

Kupfersalze werden, gewünschtenfalls mit anderen Stoffen, als Zersetzungskatalysatoren zur Beschleunigung der Zersetzung in der 1. Stufe zugegeben. Die Salze können Kupfer(I)- oder zweckmässig Kupfer(II)salze sein. Geeignete Salze sind Kupfer(II)acetat, Kupfer(II)bromid, Kupfer(II)chlorid oder die entsprechenden Kupfer(I) salze. Bevorzugt ist Kupfer(II)chlorid.

Zweckmässig sind 0,005 bis 0,05, vorzugsweise 0,01 bis 0,02 mol Kupfersalz, bezogen auf 2 mol Ausgangsstoff II oder III.

Zweckmässig wird auch Alkalijodid zusätzlich als Zersetzungskatalysator verwendet. Vorteilhaft sind Mengen von 0,005 bis 0,05 mol/mol Ausgangsstoff II oder III.

Die Reaktion kann wie folgt durchgeführt werden: Man stellt in üblicher Weise eine Diazoniumlösung her und zerstört gegebenenfalls mit Harnstoff überschüssige salpetrige Säure. Nun gibt man Metallsalz und zweckmässig Alkalijodid dazu und stellt eine Temperatur von 20 bis 50, insbesondere 30 bis 40°C, als Zersetzungstemperatur der 1. Stufe ein. Die Verweilzeiten liegen zweckmässig bei der 1. Stufe bei 1 bis 3 h, bei der 2. Stufe bei 0,1 bis 2 h. Nun wird zweckmässig Schwefeldioxid eingeleitet und die Zersetzung mit vorgenannten Komponenten und in der vorgenannten Verweilzeit in den zwei Stufen durchgeführt. Man kann während der Umsetzung in der 2. Stufe während der gesamten Reaktionszeit oder eines Teiles der Reaktionszeit zusätzliches $SO_2$ einleiten. Man kann aber auch die gesamte $SO_2$-Menge für die Umsetzung der beiden Stufen vorteilhaft schon in der 1. Stufe zugeben. In der Regel leitet man 3,5 bis 7, zweckmässig in der 1. Stufe 2 bis 6 mol Schwefeldioxid und in der 2. Stufe 0 bis 3 mol/mol Ausgangsstoff II oder III ein. In den 2 Stufen kann man die Temperatur kontinuierlich erhöhen oder in jeder Stufe die Temperatur während der ganzen oder eines Teiles der Verweilzeit in der Stufe bei einer oder bei mehreren der vorgenannten Temperaturen halten. Beispielsweise wird die Temperatur stufenweise von 20 bis auf 100°C erhöht.

Damit sich das Diazoniumsalz nicht zu heftig zersetzt, wird das Reaktionsgemisch in Stufe 1 höchstens bis 50°C erwärmt. In einer bevorzugten Ausführungsform wird in Stufe 1 ein Strom von 2 bis 4,5 mol Schwefeldioxid, bezogen auf 1 mol Ausgangsstoff II oder III, in das Reaktionsgemisch geleitet. Dann erwärmt man das Reaktionsgemisch auf 80 bis 100°C und leitet in Stufe 2 Schwefeldioxid in einer Menge von 0 bis 2 mol/mol Ausgangsstoff II oder III ein. Währenddessen scheidet sich aus der anfangs klaren Lösung in der 2. Stufe die o,o'-Dithiodibenzoesäure ab. Man führt vorteilhaft während 20 bis 55 min bei 80 bis 100°C die Umsetzung der 2. Stufe durch. Man filtriert und trocknet unter Vakuum.

In einer weiteren Ausführungsform wird die Diazoniumlösung bei gleichzeitiger Einleitung von $SO_2$ zur Kupfersalzlösung gegeben.

Die nach dem Verfahren der Erfindung erhältlichen o,o'-Dithiodibenzoesäuren sind wertvolle Ausgangsstoffe für die Herstellung von Pflanzenschutzmitteln, Farbstoffen und Pharmaka. Sie dienen zur Herstellung von Saccharinen. Bezüglich der Verwendung wird auf die US-Patentschriften Nrn. 2705242 und 2667503 verwiesen.

*Beispiel 1*

14,4 g Anthranilsäure wurden in 70 g Wasser und 18 g 30%iger Salzsäure suspendiert. Man gab unter Eiskühlung 29,4 g einer 25%igen wässerigen Natriumnitritlösung so zu, dass die Innentemperatur nicht über 5°C anstieg. Nach Zulaufende rührte man 5 min bei 0°C und zerstörte überflüssige salpetrige Säure mit 1 g Harnstoff. Nun gab man die auf 0°C gekühlte Diazoniumlösung während 1 h zu 0,4 g einer 50%igen wässerigen Lösung von Kupfer(II)chlorid · $2H_2O$ bei 35°C. Man leitete gleichzeitig mit der Diazoniumlösung einen Strom von 18,4 g $SO_2$/h in das Reaktionsgefäss, wobei die Temperatur auf 35°C gehalten wurde. Nach 60 min bei 35°C ab Reaktionsbeginn drosselte man den $SO_2$-Strom auf 16,4 g $SO_2$/h. Gleichzeitig erhitzte man während 30 min auf Rückflusstemperatur (98°C). Als man eine Temperatur von 98°C erreicht hatte, stellte man die $SO_2$-Begasung ab. Die Verweilzeit (ab Beginn der Umsetzung) in der 1. Stufe betrug insgesamt 70 min, die Verweilzeit in der 2. Stufe 40 min. 2 h nach Zersetzungsbeginn (Beginn der 1. Stufe) war die o,o'-Dithiodibenzoesäure bereits in einer Ausbeute von 90% der Theorie entstanden. Man kühlte auf 50°C, filtrierte und trocknete den Filterkuchen 2 h bei 100°C im Vakuum. Ausbeute 14,5 g vom Schmelzpunkt = Fp. 286 bis 288°C (94,8% der Theorie).

*Beispiel 2*

137,5 g Anthranilsäure wurden in 500 g Wasser und 134 g 30%iger Salzsäure gelöst und auf 5°C abgekühlt. Man setzte unter Rühren 241 g einer 30%igen $NaNO_2$-Lösung so zu, dass die Innentemperatur nicht über 5°C anstieg. In einem Rührgefäss hatte man 6 g einer 50%igen wässerigen Lösung von $Cu(II)Cl_2 \cdot 2H_2O$ sowie 1,6 g Kaliumjodid in 100 g Wasser vorgelegt. Über eine Pumpe förderte man innerhalb 1 h die zuvor hergestellte Lösung des Diazoniumsalzes der Anthranilsäure in das Zersetzungsgefäss. Von Beginn der Zugabe von Diazoniumlösung an leitete man einen Strom von 184 g $SO_2$/h in das Zersetzungsgefäss. Dessen Innentemperatur wurde von Beginn an durch Kühlung auf 30°C gehalten. Nachdem alle Diazoniumsalzlösung eingebracht war (60 min), drosselte man den $SO_2$-Strom auf 130 g/h. Nach 90 min (ab Beginn) stellte man den $SO_2$-Strom ab. Dann erhitzte man innerhalb 30 min auf Rückflusstemperatur (99°C). Die Verweilzeit (ab Beginn der Umsetzung) in der 1. Stufe betrug insgesamt 1,7 h, in der 2. Stufe 0,7 h. Man hielt das Gemisch 20 min bei 99°C, kühlte dann während 20 min auf 40°C und filtrierte. Man erhielt 143,8 g (94% der Theorie) o,o'-Dithiobenzoesäure vom Schmelzpunkt = Fp. 286-288°C.

## Patentanspruch

Verfahren zur Herstellung von o,o'-Dithiodibenzoesäuren der Formel

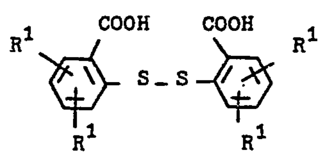
(I)

worin die einzelnen Reste $R^1$ gleich oder verschieden sein können und jeweils ein Wasserstoffatom, ein Halogenatom, eine Nitrogruppe, eine Gruppe $-SO_3H$ oder einen aliphatischen Rest bedeuten, durch Umsetzung von diazotierten Anthranilsäurederivaten mit Schwefeldioxid in Gegenwart von Säure, Wasser und Katalysatoren zur Zerstörung der Diazoniumsalze, dadurch gekennzeichnet, dass man ein Diazoniumsalz von Anthranilsäuren der Formel

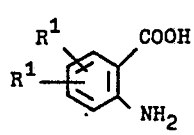
(II)

oder ein Diazoniumsalz von Anthranilsäureestern der Formel

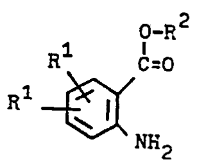
(III)

worin $R^1$ die vorgenannte Bedeutung besitzt und $R^2$ einen aliphatischen Rest bedeutet, in Gegenwart eines Kupfersalzes und gewünschtenfalls in Gegenwart von Alkalijodid in einer 1. Stufe bei einer Temperatur von 20 bis 50°C und dann in einer 2. Stufe bei einer Temperatur von 80 bis 100°C umsetzt.

## Claim

A process for the preparation of o,o'-dithiodibenzoic acid of the formula

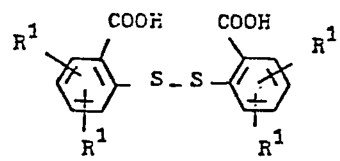
(I)

where the individual radicals $R^1$ may be identical or different and are each hydrogen, halogen, nitro, $-S=O_3H$ or an aliphatic radical, by reaction of a diazotized anthranilic acid derivative with sulfur dioxide in the presence of an acid, water and a catalyst for destroying the diazonium salt, wherein a diazonium salt of an anthranilic acid of the formula

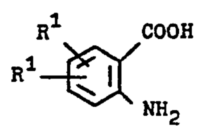
(II)

or a diazonium salt of an anthranilate of the formula

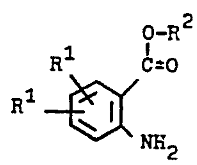
(III)

where $R^1$ has the above meanings and $R^2$ is an aliphatic radical, is reacted, in the presence of a copper salt and, if desired, in the presence of an alkali metal iodide, in a 1st stage at from 20 to 50°C and then in a 2nd stage at from 80 to 100°C.

## Revendication

Procédé de préparation d'acides o,o'-dithiodibenzoïques répondant à la formule

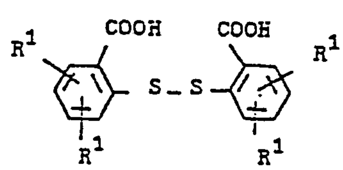
(I)

dans laquelle les différents restes $R^1$ peuvent être identiques ou différents et représentent respectivement un atome d'hydrogène, un atome d'halogène, un groupe nitro, un groupe $-SO_3H$ ou un reste aliphatique, en faisant réagir des dérivés diazotés de l'acide anthranilique sur de l'anhydride sulfureux en présence d'acide, d'eau et de catalyseurs de décomposition des sels de diazonium, caractérisé en ce qu'on fait réagir un sel de

diazonium d'acides anthraniliques répondant à la formule

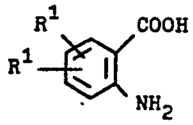

(II)

ou un sel de diazonium d'esters anthraniliques répondant à la formule

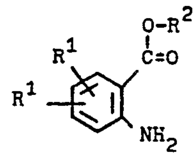

(III)

dans laquelle $R^1$ a la signification précédente et $R^2$ représente un reste aliphatique, en présence d'un sel de cuivre et, si on le souhaite, d'iodure alcalin, au cours d'un premier stade à une température de 20 à 50° C, puis, au cours d'un second stade, à une température de 80 à 100° C.